(19) Europäisches Patentamt
European Patent Office
Office européen des brevets

(11) Veröffentlichungsnummer: **0 000 496**
**B1**

(12) **EUROPÄISCHE PATENTSCHRIFT**

(45) Veröffentlichungstag der Patentschrift: **19.05.82**

(51) Int. Cl.³: **C 08 F 20/34**, C 08 K 5/34

(21) Anmeldenummer: **78100360.3**

(22) Anmeldetag: **11.07.78**

# (54) Seitenständige N-heterocyclische Ringe tragende Acrylatpolymere und deren Verwendung als Lichtschutzmittel.

(30) Priorität: **19.07.77 CH 8915/77**

(43) Veröffentlichungstag der Anmeldung:
**07.02.79 Patentblatt 79/3**

(45) Bekanntmachung des Hinweises auf die Patenterteilung:
**19.05.82 Patentblatt 82/20**

(84) Benannte Vertragsstaaten:
**BE CH DE FR GB NL SE**

(56) Entgegenhaltungen:
**DE - A - 2 623 464**
**GB - A - 1 112 439**
**US - A - 3 590 045**
**US - A - 3 705 166**

(73) Patentinhaber: **CIBA-GEIGY AG**
**Patentabteilung Postfach**
**CH-4002 Basel (CH)**

(72) Erfinder: **Karrer, Friedrich, Dr.**
**Rebbergstrasse 5**
**CH-4800 Zofingen (CH)**

**EP 0 000 496 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

Seitenständige N-heterocyclische Ringe tragende Acrylatpolymere und deren Verwendung als Lichtschutzmittel

Die vorliegende Erfindung betrifft seitenständige N-heterocyclische Ringe tragende Acrylat-Homo- und Copolymere sowie deren Verwendung als Lichtschutzmittel in organischen Polymeren.

Aus dem US Pat. No. 3,705,166 ist es bekannt, monomere Acrylsäurederivate, welche mindestens eine Piperidinylgruppe mit einem sterisch gehinderten Stickstoffatom enthalten, als Lichtschutzmittel in organischen Polymeren zu verwenden. Diese Acrylsäurederivate sind allerdings zu leicht flüchtig. Ferner wird auf die Möglichkeit hingewiesen, das monomere Additiv in gewisse Substrate einzubauen. Dies hat allerdings den Nachteil, dass die Polymerstruktur durch das eingebaute Additiv gestört ist, was zu schwer kontrollierbarer Veränderung der Eigenschaften des zu schützenden Substrats führen kann. In der JP—OS No. 49—75469 sind wasserlösliche Polymere Acryl- und Methacrylsäurederivate als Flockulierungsmittel vorgeschlagen worden.

Es wurden nun homo- und copolymere Additive gefunden, welche neben ausgezeichneten lichtstabilisierenden Eigenschaften, gute Löslichkeit bzw. Verträglichkeit im Polymer und eine hohe Extraktionsbeständigkeit aufweisen.

Die vorliegende Erfindung betrifft somit organische Polymere, enthaltend als Lichtschutzmittel seitenständige N-heterocyclische Ringe tragende homopolymere Verbindungen mit der wiederkehrenden Struktureinheit der Formel I

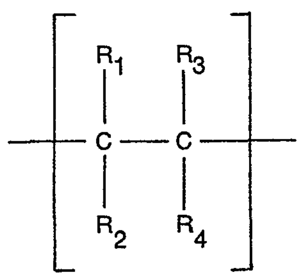

(I),

worin

$R_1$ eine einen N-heterocyclischen Ring enthaltende Gruppe der Formeln II, III, IV und V

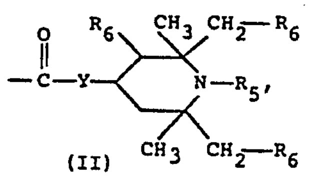

(II)

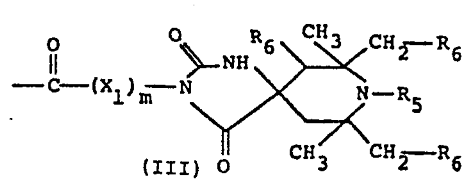

(III)

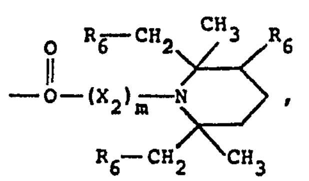

(IV)

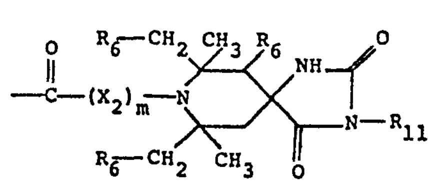

(V)

bedeuten, worin

$R_5$ Wasserstoff, Oxyl-Sauerstoff, $C_1$—$C_{18}$ Alkyl, $C_3$—$C_8$ Alkenyl, $C_3$—$C_6$ Alkinyl, $C_7$—$C_{12}$ Aralkyl, $C_2$—$C_{21}$ Alkoxyalkyl, eine aliphatische Acylgruppe mit 1—4 C-Atomen oder eine der Gruppen $-CH_2COOR_7$ oder $-COOR_8$ ist, wobei

$R_7$ $C_1$—$C_{12}$ Alkyl, $C_3$—$C_8$ Alkenyl, Phenyl, $C_7$—$C_8$ Aralkyl oder Cyclohexyl bedeutet, und

$R_8$ $C_1$—$C_{12}$ Alkyl, Phenyl, Benzyl oder Cyclohexyl ist, und

$R_6$ Wasserstoff oder $C_1$—$C_4$ Alkyl bedeutet, und

Y $-O-$ oder $-\overset{|}{N}-R_9$ ist, wobei

$R_9$ Wasserstoff, $C_1$—$C_{12}$ Alkyl, $C_7$—$C_{12}$ Aralkyl oder Cyclohexyl ist, und

$X_1$ eine Gruppe der Formel $-O-CH(R_{10})-CH_2-$ (VI) ist, worin

$R_{10}$ Wasserstoff, Methyl, Aethyl, Phenoxymethyl oder Phenyl bedeutet, und

n 0 oder 1 ist, und

$X_2$ eine Gruppe der Formel VI, worin $R_{10}$ die oben angegebene Bedeutung hat, oder eine Gruppe der Formel $—O—CH_2—CH(OH)—CH_2—$ (VII) ist, und
m 0 oder 1 ist, und
$R_{11}$ $C_1—C_{18}$ Alkyl, gegebenenfalls durch $C_1—C_4$ Alkyl oder $C_1—C_4$ Alkoxy substituiertes Cyclohexyl, Phenyl oder Benzyl bedeutet, und
$R_2$ Wasserstoff oder $C_1—C_4$ Alkyl ist, und
$R_1$ und
$R_3$ falls $R_2$ und $R_4$ Wasserstoff sind, zusammen eine Gruppe der Formel VIII

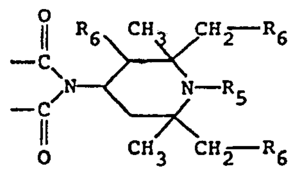

(VIII) bilden, worin
$R_5$ und
$R_6$ die oben angegebene Bedeutung haben, und

$R_3$ Wasserstoff oder $C_1—C_2$ Alkyl ist, und
$R_4$ Wasserstoff oder Methyl bedeutet,
oder deren Copolymere mit mindestens eine polymerisierbare Doppelbindung enthaltenden Verbindungen, wobei das Molverhältnis der Komponente der Formel I zur Co-Monomeren-Komponente bis zu 1:10 beträgt und worin das Molekulargewichts-Zahlenmittel der homo- oder copolymeren Lichtschutzmittel 500 bis 150'000, vorzugsweise 500 bis 50'000 beträgt.

$R_1$ kann eine Gruppe der Formel II, III, IV oder V sein. Bevorzugt ist $R_1$ eine Gruppe der Formel II oder IV.

$R_2$ bedeutet als $C_1—C_4$ Alkyl z.B. Methyl, Aethyl, n-Propyl oder n-Butyl, insbesondere Aethyl, vor allem aber Methyl oder Wasserstoff.

$R_3$ bedeutet als $C_1—C_2$ Alkyl, Aethyl oder insbesondere Methyl.

$R_5$ ist als $C_1—C_{18}$ Alkyl z.B. Methyl, Aethyl, n-Propyl, n-Butyl, n-Pentyl, n-Octyl, n-Decyl, n-Dodecyl oder Octadecyl. Bevorzugt sind Alkylgruppen mit 1—12, ferner solche mit 1—8, insbesondere solche mit 1—4 C-Atomen und vor allem Methyl.

$R_5$ ist als $C_3—C_8$ Alkenyl beispielsweise Allyl, 3-Methyl-2-butenyl, 2-Butenyl, 2-Hexenyl oder 2-Octenyl, insbesondere Allyl.

$R_5$ ist als $C_3—C_6$ Alkinyl z.B. Propargyl.

$R_5$ ist als $C_7—C_{12}$ Aralkyl z.B. Benzyl, $\beta$-Phenyl-äthyl oder 4-tert.-Butyl-benzyl.

Bedeutet $R_5$ $C_2—C_{21}$ Alkoxyalkyl, so kann der Alkylteil 1—3 C-Atome enthalten und der Alkoxy-Teil aus 1—18 C-Atomen bestehen, wie z.B. in Methoxymethyl, Aethoxymethyl, 2-Methoxyäthyl, 2-Aethoxyäthyl, 2-n-Butoxyäthyl, 3-n-Butoxypropyl, 2-Octoxyäthyl oder 2-Octadecyloxyäthyl; insbesondere zu erwähnen sind Verbindungen, in denen $R_5$ eine Alkoxyalkylgruppe mit 2—6 C-Atomen bedeutet.

$R_5$ ist als aliphatische Acylgruppe mit 1—4 C-Atomen, beispielsweise Formyl, Acetyl, Acryloyl oder Crotonoyl, insbesondere Acetyl.

$R_6$ ist als $C_1—C_4$ Alkyl verzweigtes oder insbesondere unverzweigtes Alkyl, wie Aethyl, n-Propyl oder n-Butyl, vor allem aber Methyl. Bevorzugt ist $R_6$ Wasserstoff.

$R_9$ ist als $C_1—C_{12}$ Alkyl z.B. Methyl, Aethyl, n-Propyl, n-Butyl, n-Pentyl, n-Octyl, n-Decyl oder n-Dodecyl, bevorzugt jedoch $C_1—C_4$ Alkyl.

$R_9$ ist als $C_7—C_{12}$ Aralkyl insbesondere Phenyläthyl oder vor allem Benzyl.

$R_{10}$ ist Phenyl oder Phenoxymethyl, bevorzugt Methyl, Aethyl und insbesondere Wasserstoff.

$R_{11}$ ist als $C_1—C_{18}$ Alkyl z.B. Methyl, Aethyl, Propyl, Butyl, Hexyl, Octyl, Dodecyl oder Octadecyl, Bevorzugte Alkylgruppen sind solche mit 1—12 C-Atomen.

$R_{11}$ kann auch Benzyl, Cyclohexyl oder Phenyl bedeuten, welche im Kern mit $C_1—C_4$ Alkyl, wie Methyl, Aethyl, Propyl oder n-Butyl oder mit $C_1—C_4$ Alkoxy, wie Methoxy, Aethoxy, Propoxy oder n-Butoxy substituiert sein können.

Ist $R_5$ die Gruppe $—CH_2COOR_7$ oder $—COOR_8$, so bedeuten $R_7$ und $R_8$ als $C_1—C_{12}$ Alkyl, z.B. Methyl, Aethyl, Isopropyl, n-Butyl, Isobutyl, t-Butyl, Isopentyl, n-Octyl oder n-Dodecyl, Bevorzugt sind $R_7$ und $R_8$ $C_1—C_4$ Alkyl. $R_7$ ist als $C_3—C_8$ Alkenyl z.B. Allyl, 2-Butenyl oder 2-Hexenyl. $R_7$ ist als $C_7—C_8$ Aralkyl z.B. Benzyl oder $\alpha$-Phenyläthyl. Y bedeutet —O— oder

$$\begin{array}{c} | \\ —NR_9— \end{array}$$

bevorzugt —O—.

Die mit der oben angegebenen Definition charakterisierten Homopolymeren stellen gute Lichtstabilisatoren dar. Dennoch ist es in vielen Fällen von Vorteil, diese Homopolymeren durch Copolymerisation mit mindestens einer Verbindung, welche eine oder mehrere polymerisierbare Doppelbindung besitzen, zu modifizieren. Dadurch kann auf einfache Weise ein Einfluss auf die Eigenschaften, wie die Löslichkeit oder den Erweichungspunkt des Additivs genommen werden. Als copolymerisierbare Komponenten eignen sich beispielsweise Styrol, Divinylbenzol, 2- oder 4-Vinylpyridin, Verbindungen aus der Acrylsäurereihe, wie Ester oder Amide, die sich von Acrylsäure oder Methacrylsäure ableiten, z.B. Methylacrylat, Butylacrylat, Methylmethacrylat, Acrylnitril, Methacrylnitril, Acrylsäureglycidylester, Methylenbisacrylamid oder Aethylenglykoldimethylacrylat; oder es kann sich um 1-Alkene mit 2—10 C-Atomen, z.B. Aethylen, Propylen, 1-Buten, 1-Penten, 1-Hexen, 1-Octen oder 1-Decen handeln; ferner kann es sich um Isopren, Butadien, Vinylester, wie Essigsäurevinylester, um Vinyläther, N-Vinylpyrrolidon-2, N-Vinylcarbazol, Maleinimide oder ungesättigte Phosphonate handeln. Bevorzugte copolymerisierbare Komponenten sind Styrol, Acrylnitril, Acryl- oder Methacrylsäureester, Vinylester, Vinyläther oder Acryl- bzw. Methacrylamide.

Es können auch Copolymere aus zwei verschiedenen Piperidyl-Monomeren hergestellt werden. Verbindungen in welchen $R_1$ und $R_2$ zusammen eine Gruppe der Formel VIII bedeutet, eignen sich insbesondere für Copolymerisationsreaktionen, sei es mit anderen Piperidinyl-Monomeren, oder anderen oben erwähnten polimerisierbaren Komponenten.

Die spezifisch als Co-Monomer-Komponente gewählte Verbindung spielt eine untergeordnete Rolle, d.h. es besteht eine weitgehende Austauschbarkeit der oben aufgelisteten Verbindungen. Die Massnahmen, welche ergriffen werden müssen, um zu Copolymeren ähnlichen Molekulargewichts und ähnlicher Eigenschaften zu kommen, sind dem Fachmann bekannt. Selbstverständlich können auch Copolymere aus mehr als zwei monomeren Komponenten, z.B. Terpolymere verwendet werden.

Das Molekulargewicht der erfindungsgemässen Homo- bzw. Copolymeren beträgt mehr als 500; es kann aber durchaus bis zu 150'000 ausmachen. Bevorzugte Verbindungen besitzen ein Molekulargewicht von 500 bis 50'000 und insbesondere 1000—20'000.

Wie bereits erwähnt, kann die copolymerisierbare Komponente völlig fehlen oder bis zu einem 10-fachen Ueberschuss betragen. Das Molverhältnis der seitenständig N-heterocyclische Ring enthaltenden Verbindung zur Co-Komponente beträgt also bevorzugt ungefähr 1:0,001 bis 1:10, insbesondere 1:0,001 bis 1:5. Dies bedeutet also, dass die Menge der Co-Komponente (sehr kleine sein kann (z.B. 0,1% bezogen auf die Komponente der Formel I). Dies wird insbesondere der Fall sein, wenn die Co-Komponente zwei polymerisierbare Doppelbindungen besitzt, wie Divinylbenzol oder Bisacrylate bzw. Bismethacrylate, mit welchen verzweigte oder vernetzte Additive entstehen.

Hervorzuheben sind homopolymere Verbindungen der Formel I, worin

$R_1$ eine Gruppe der Formel II, III, IV oder V bedeutet worin
$R_5$ Wasserstoff, $C_1$—$C_{12}$ Alkyl, $C_3$—$C_5$ Alkenyl, Propargyl, $C_7$—$C_8$ Aralkyl, Acetyl oder $C_2$—$C_{10}$ Alkoxyalkyl bedeutet, und
$R_6$ Wasserstoff oder Methyl bedeutet, und
Y —O— oder

$$-\overset{|}{N}-R_9$$

ist, worin
$R_9$ Wasserstoff, $C_1$—$C_{12}$ Alkyl oder Benzyl ist, und
$X_1$ eine Gruppe der Formel VI, worin $R_{10}$ Wasserstoff oder Methyl ist, bedeutet, und
n 0 oder 1 ist, und
$X_2$ eine Gruppe der Formeln VI oder VII ist, worin $R_{10}$ die oben angegebene Bedeutung hat, und
m 0 oder 1 ist, und
$R_{11}$ $C_1$—$C_{12}$ Alkyl oder Benzyl ist, und
$R_2$ Wasserstoff oder $C_1$—$C_2$ Alkyl bedeutet, oder
$R_1$ und
$R_3$ falls $R_2$ und $R_4$ Wasserstoff sind, zusammen eine Gruppe der Formel VIII bilden, worin $R_5$ und $R_6$ die oben angegebene Bedeutung haben, und

$R_3$ Wasserstoff oder $C_1$—$C_2$ Alkyl ist, und
$R_4$ Wasserstoff oder Methyl ist,
sowie deren Copolymere mit mindestens einer polymerisierbare Doppelbindung enthaltenden Verbindungen.

Bevorzugt sind homopolymere Verbindungen der Formel I, worin

$R_1$ eine Gruppe der Formeln II oder IV bedeutet, worin
$R_5$ Wasserstoff, $C_1$—$C_8$ Alkyl, Benzyl oder Acetyl ist,

$R_6$ Wasserstoff oder Methyl ist, und
Y —O— oder

$$-\overset{|}{N}-R_9$$

bedeutet, worin
$R_9$ Wasserstoff oder $C_1$—$C_8$ Alkyl ist, und
$X_2$ eine Gruppe der Formeln VI oder VII ist, worin
$R_{10}$ Wasserstoff, Methyl oder Aethyl ist, und
m 0 oder 1 ist, und
$R_2$ Wasserstoff oder Methyl bedeutet, oder
$R_1$ und
falls $R_2$ und $R_4$ Wasserstoff sind, zusammen eine Gruppe der Formel VIII sind, worin $R_5$ und $R_6$ die oben angegebene Bedeutung haben, und

$R_3$ Wasserstoff oder Methyl ist, und
$R_4$ Wasserstoff bedeutet,
sowie deren Copolymere mit mindestens eine polymerisierbare Doppelbindung enthaltenden Verbindungen.

Bevorzugt sind auch homopolymere Verbindungen der Formel I, worin

$R_1$ eine Gruppe der Formeln II oder IV bedeutet, worin
$R_5$ Wasserstoff, $C_1$—$C_4$ Alkyl, Benzyl oder Acetyl bedeutet, und
$R_6$ Wasserstoff ist, und
Y —O— oder

$$-\overset{|}{N}-R_9$$

bedeutet, worin
$R_9$ Wasserstoff oder $C_1$—$C_4$ Alkyl ist, und
$X_2$ eine Gruppe der Formeln VI oder VII ist, worin
$R_{10}$ Wasserstoff oder Methyl ist, und
m 0 oder 1 ist, und
$R_2$ und
$R_3$ unabhängig voneinander Wasserstoff oder Methyl sind und

$R_4$ Wasserstoff bedeutet,
sowie deren Copolymere mit Styrol, Acrylnitril, einem Acryl- oder einem Methacrylsäureester, einem Vinylester, einem Vinyläther oder einem Acryl- oder Methacrylamid.

Besonders bevorzugt sind homopolymere Verbindungen der Formel I, worin

$R_1$ eine Gruppe der Formeln II oder IV bedeutet, worin
$R_5$ Wasserstoff, Methyl oder Acetyl ist, und
$R_6$ Wasserstoff ist, und
Y —O— oder

$$-\overset{|}{N}-R_9$$

ist, worin
$R_9$ Wasserstoff oder $C_1$—$C_4$ Alkyl bedeutet, und
$X_2$ eine Gruppe der Formeln VI oder VII ist, worin
$R_{10}$ Wasserstoff ist, und
m 0 oder 1 ist, und
$R_3$ und
$R_4$ Wasserstoff bedeuten, und

$R_2$ Wasserstoff oder Methyl ist,
sowie deren Copolymere mit Styrol, Acrylnitril, einem Acryl- oder Methacrylsäureester, einem Vinylester, einem Vinyläther, oder einem Acryl- oder Methacrylamid.

Beispiele von Monomeren, die sich für die Polymerisation bzw. Copolymerisation zu den erfindungsgemässen Polymeren eignen sind:

—1-Methacryloyloxyäthyl-2,2,6,6-tetramethyl-piperidin
—1-Acryloyloxyäthyl-2,2,6,6-tetramethyl-piperidin
—1,2,2,6,6-Pentamethyl-4-maleinimido-piperidin
—1-Acetyl-2,2,6,6-tetramethyl-4-maleinimido-piperidin
—1-Benzyl-2,2,6,6-tetramethyl-4-maleinimido-piperidin
—1,3,8-Triaza-2,4-dioxo-3-acryloyl-oxyäthyl-7,7,8,9,9-pentamethyl-spiro-[4,5]-decan
—1,3,8-Triaza-2,4-dioxo-3-methacryloyl-oxyäthyl-7,7,8,9,9-pentamethyl-spiro-[4,5]-decan
—1,3,8-Triaza-2,4-dioxo-3-n-dodecyl-7,7,9,9-tetramethyl-8-methacryloyl-oxyäthyl-spiro-[4,5]-decan
—1,3,8-Triaza-2,4-dioxo-3-methacryloyl-oxyäthyl-7,7,9,9-tetramethyl-8-benzyl-spiro-[4,5]-decan
—1,3,8-Triaza-2,4-dioxo-3-n-butyl-7,7,9,9-tetramethyl-8-acryloyl-oxyäthyl-spiro-[4,5]-decan
—1-Benzyl-2,2,6,6-tetramethyl-4-(N-n-butyl)-methacrylamido-piperidin
—1,2,2,6,6-Pentamethyl-4-(N-benzyl)-acrylamido-piperidin
—1,2,2,6,6-Pentamethyl-4-(N-n-propyl)-acrylamido-piperidin
—1,2,2,6,6-Pentamethyl-4-(N-n-propyl)-methacrylamido-piperidin
—1-Allyl-2,2,6,6-tetramethyl-4-acryloyloxy-piperidin
—1-Allyl-2,2,6,6-tetramethyl-4-methacryloyloxy-piperidin
—1,2,2,6,6-Pentamethyl-4-acrylamido-piperidin
—1-Benzyl-2,2,6,6-tetramethyl-4-(N-n-butyl)-acrylamido-piperidin
—1-Benzyl-2,2,6,6-tetramethyl-4-acrylamido-piperidin
—1-[3'-Acryloyloxy-(2'-hydroxy)-propyl]-2,2,6,6-tetramethyl-piperidin.

Die Monomeren, welche sich als Ausgangsstoffe für die Polymerisationsreaktion eignen entsprechen der allgemeinen Formel IX

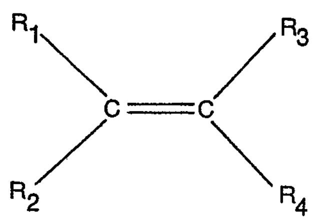

(IX),

worin $R_1$, $R_2$, $R_3$ und $R_4$ die oben definierte Bedeutung haben. Mit Ausnahme der Verbindungen, in welchen $R_1$ eine Gruppe der Formel II bedeutet, worin Y —O—, oder —NH, und $R_5$ und $R_6$ Wasserstoff ist, sowie $R_2$ und $R_3$ Wasserstoff oder Methyl und $R_4$ Wasserstoff bedeuten, sind die Monomeren neu und stellen daher auch einen Gegenstand der Erfindung dar. Neben ihrer Nützlichkeit als Ausgangsstoffe bei der Herstellung von homo- oder copolymeren Lichtschutzmitteln sind die Monomeren der Formel IX selbst Lichtschutzmittel für organisches Material.

Die Herstellung der zur Polymerisation eingesetzten Monomere der Formel IX erfolgt auf an sich bekannte Weise, z.B. analog der im US Pat. No. 3,705,166 beschriebenen Methoden. Es wird dabei beispielsweise ein reaktionsfähiges Derivat einer ungesättigten Carbonsäure der Formel X

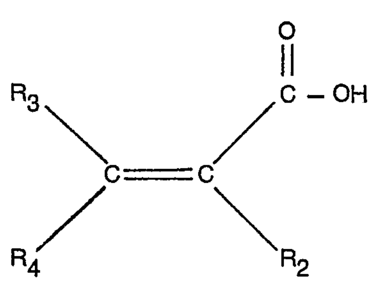

(X)

mit einer der Verbindungen der Formeln XI, XII, XIII oder XIV

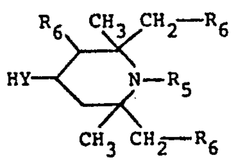

(XI)

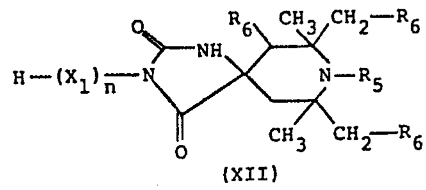

(XII)

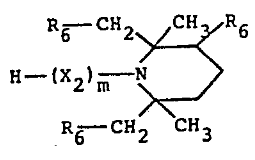

(XIII)

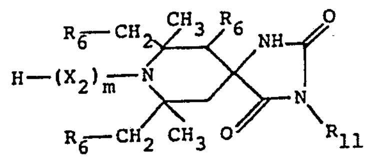

(XIV)

worin alle Substituenten oben definierte Bedeutung haben, vorzugsweise in einem inerten organischen Lösungsmittel umgesetzt.

Reaktionsfähige Derivate einer ungesättigten Carbonsäure sind beispielsweise ein Säurehalogenid der Formel Xa

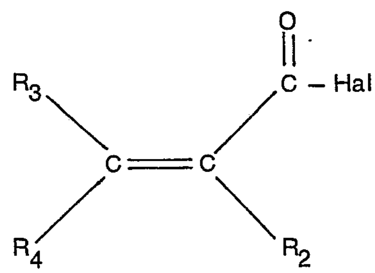

(Xa),

worin Hal, Brom oder insbesondere Chlor bedeutet, oder ein Säureanhydrid der Formel Xb

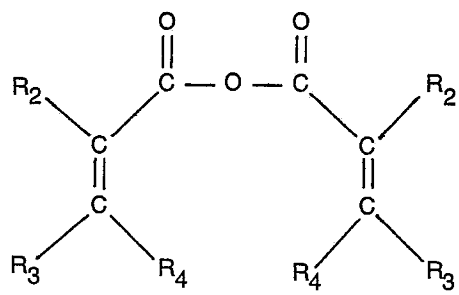

(Xb)

Pro Mol einer der Verbindungen der Formeln XI, XII, XIII oder XIV wird vorzugsweise ungefähr ein Mol einer Verbindung der Formel Xa oder Xb eingesetzt. $R_2$, $R_3$ und $R_4$ in den Formeln Xa und Xb entsprechen der oben angegebenen Definition.

Beim Verwenden eines Säurehalogenids der Formel Xa arbeitet man in Gegenwart einer Base z.B. in Gegenwart eines tert.Amins, wie Triäthylamin, Di-isopropyl-aethylamin, N,N-Diäthylanilin oder Pyridin; oder in Gegenwart eines wasserfreien Alkalimetall- oder Erdalkalimetallcarbonats, oder Alkalimetallbicarbonats, wie $MgCO_3$, $NaHCO_3$, $Na_2CO_3$ oder $K_2CO_3$. Wird anstelle des Säurechlorids ein Säureanhydrid der Formel Xb verwendet, so kann gegebenenfalls auf die Base verzichtet werden.

Die bei den oben beschriebenen Verfahrensvarianten verwendeten organischen Lösungsmittel müssen gegenüber den Reaktionspartnern inert sein. Es eignen sich dabei beispielsweise aliphatische Kohlenwasserstoffe wie Hexan oder Ligroin; aromatische Kohlenwasserstoffe, wie Benzol, Toluol oder Xylol; chlorierte Kohlenwasserstoffe, wie Methylenchlorid oder Chloroform; Amide, wie Hexamethylenphosphorsäuretriamid; oder Aether wie Dioxan, 1,2-Dimethoxyäthan, Diäthyläther oder Tetrahydrofuran.

Die Temperatur dieser Reaktion beträgt vorzugsweise –20 bis +120°C, insbesondere aber –10°C bis +80°C.

Die Verbindungen der Formel I, in welchen $R_1$ und $R_3$ zusammen eine Gruppe der Formel VIII bilden, werden analog dem oben beschriebenen Säureanhydrid-Verfahren hergestellt, wobei ungefähr ein Mol Maleinsäureanhydrid mit ungefähr einem Mol einer Verbindung der Formel XI, worin Y

|
—NH

ist, und $R_5$ und $R_6$ oben definierte Bedeutung haben, umgesetzt. Das dabei entstehende Amidsäurezwischenprodukt wird anschliessend mit einem wasserbindenden Mittel, z.B. Essigsäureanhydrid behandelt, wobei unter Ringschluss Wasser abgespalten wird.

Eine weitere Verfahrensvariante besteht darin, dass man als reaktionsfähiges Derivat einer Carbonsäure der Formel X einen Ester der Formel Xc

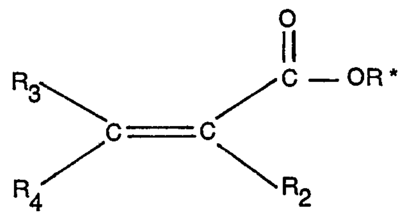

(Xc)

worin $R_2$, $R_3$ und $R_4$ die oben angegebene Bedeutung haben und R* $C_1$—$C_4$ Alkyl ist, verwendet. Die Reaktionspartner der Formeln XI, XII, XIII oder XIV, worin m und n 1 sind und die übrigen Symbole die oben angegebene Bedeutung haben, können in ungefähr stöchiometrischen Mengen oder mit einem Ueberschuss an Xc zur Reaktion gebracht werden.

Es handelt sich dabei um eine übliche Umesterungsmethode, welche bei erhöhter Temperatur, mit oder ohne Lösungsmittel und in Gegenwart eines Umesterungskatalysators, z.B. einer Säure oder vorzugsweise einer Base stattfindet. Die Temperatur beträgt vorzugsweise 20—170°C, insbesondere 50—150°C. Wird in einem Lösungsmittel gearbeitet, so kann eines der oben angeführten verwendet werden. Es kann auch ein Ionenaustauschharz als Katalysator eingesetzt werden. Da es sich bei den Verbindungen der Formel Xc um flüssige Verbindungen handelt, kann gegebenenfalls auf ein Lösungsmittel verzichtet werden. Die Reaktionskomponente Xc ist gegebenenfalls vor der Umesterungsreaktion mit einem der bekannten Stabilisatoren, wie z.B. Hydrochinon, Hydrochinonmonomethyläther, 2,6-Di-tert.-butyl-p-cresol, einem anderen 2,6-Di-tert.-butyl-phenyl-Derivat oder Phenothiazin, zu stabilisieren.

Die als Ausgangsstoffe verwendeten Verbindungen der Formeln X, Xa, Xb und Xc sind dem Fachmann bekannte Verbindungen und können, falls nicht im Handel erhältlich auf einfache Weise hergestellt werden.

Die als Reaktionspartner verwendeten Piperidinylderivate sind ebenfalls bekannte Verbindungen. Die Herstellung der Verbindungen der Formel XI, ist beispielsweise der DT—OS 2,352,658 (4-Hydroxypiperidine) oder in dem US Pat. No. 3,684,765 (4-Aminopiperidine) beschrieben worden.

Die Herstellung der Verbindungen der Formeln XII und XIV kann analog der in der DT—OS 2.227.689 beschriebenen Methoden erfolgen.

Die Herstellung von Verbindungen der Formel XIII ist beispielsweise aus der DT—OS 2,418, 540 bekannt.

Die Verbindungen der Formel XI, XII, XIII und XIV, welche im Piperidylring in 2- und 6-Stellung verschiedenartige Substituenten besitzen, können durch Umsetzung eines Ketones der Formel $CH_3$—CO—$CH_2$—$R_6$ mit Ammoniak hergestellt werden. Das gebildete Pyrimidin wird, wie in Helv. Chim. Acta *30*, 114 (1947) beschrieben, zu einem Aminoketon der Formel XV hydrolysiert.

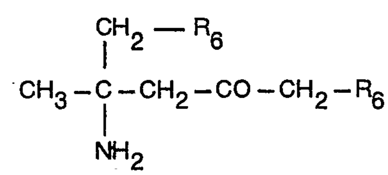

(XV)

Die Verbindungen der Formel XV werden in einem zweiten Verfahrensschritt mit Ammoniak und einem Keton $CH_3$—CO—$CH_2$—$R_6$ umgesetzt, wie es z.B. in Monatsh. Chemie *88*, 464 (1957) beschrieben ist ($R_6$ hat in den angegebenen Formeln die oben genannte Bedeutung). Die Verbindungen der Formeln XI und XII, worin $R_5$ Wasserstoff bedeutet, können aus dem so erhaltenen Pyrimidin durch Hydrolyse gewonnen werden.

Die Verbindungen, welche in 1- und/oder 4-Stellung von Wasserstoff verschiedene Substituenten tragen, werden analog der in den oben angegeben Literaturstellen beschriebenen Methoden hergestellt.

Die Monomeren der Formel IX können nach bekannten Methoden polymerisiert werden, welche beispielsweise in Houben-Weyl, *14* (1) 1010—1078 (1962) beschrieben sind.

Es eignen sich dabei vor allem die unter den Namen radikalische und ionische Homo- bzw. Copolymerisation bekannten Reaktionen. Die Polymerisation wird in bekannter Weise durch Initiatoren und Regler oder Kettenabbrecher gesteuert. Dadurch ist es möglich, zu Polymeren gewünschten Molekulargewichts zu gelangen. Die Polyreaktion kann in Substanz, in Lösung, in Dispersion, in Emulsion, in Suspension, oder als sogenannte Perlpolymerisation durchgeführt werden.

Für die radikalische Homo- bzw. Copolymerisation eignen sich vor allem Perverbindungen. Azoverbindungen sowie Redoxsysteme als Initiatoren. Gebräuchliche organische oder anorganische Perverbindungen sind u.a. Hydroperoxide, Dialkylperoxide, Diacylperoxide, Perester oder Peroxodisulfate. Beispiele für Perverbindungen sind Wasserstoffperoxid, Kaliumperoxodisulfat. Cumolhydroperoxid, Di-t-butyl-peroxid, Aethylmethylketonperoxid, Cyclohexanonperoxid oder gegebenenfalls durch Chlor oder Brom substituiertes Dibenzoylperoxid. Als Azoverbindungen eignen sich insbesondere solche, bei denen die Azogruppe beiderseits an tertiäre C-Atome gebunden ist, die neben Alkylgruppen noch Nitril- oder Estergruppen tragen, $\alpha,\alpha'$-Azoisobuttersäuredinitril und Perbenzoesäure-tert.-butylester sind wichtige Vertreter dieser Initiatorenklasse. Wird die Polyreaktion mittels eines Redoxsystems ausgelöst, so eignen sich als Oxidationsmittel organische oder anorganische Perverbindungen und als Reduktionsmittel entweder Metallionen niedriger Wertigkeitsstufe oder metallfreie Verbindungen, die sich leicht oxydieren lassen. Beispiele für Oxidationsmittel sind Wasserstoffperoxid. Peroxidisulfate oder Diacylperoxide. Als Reduktionsmittel kommen $Ag^+$, $Fe^{2+}$, $Ti^{3+}$, Hydrogensulfit, Sulfit, Thiosulfat, Mercaptane, Sulfine, Amine, Endiole (Zucker), Benzoin-$Fe^{2+}$ oder Hydrogensulfit/$Fe^{2+}$ in Frage. Während bei den Perverbindungen und den Azoverbindungen die Art des Initiators lediglich die Polymerisationsgeschwindigkeit, den mittleren Polymerisationsgrad, die Art der Endgruppen oder die Zahl der Verzweigungen, nicht aber die Polymerisierbarkeit beeinflusst, ist nicht jedes Redoxsystem für jede ungesättigte Verbindung geeignet.

Das Molekulargewicht des Polymers steuert man am einfachsten mittels geeigneter Regler. Beispiele sind Mercaptane, wie n-Butylmercaptan oder Dodecylmercaptan und andere organische Schwefelverbindungen, wie Diisopropylxanthogendisulfid, sowie aliphatische Aldehyde und Acetale oder Allylverbindungen, wie Allylalkohol. Die Reaktions-Temperaturen sind dem Fachmann bekannt und betragen für die radikalische Polymerisation je nach Art der verwendeten Komponenten —20°C bis +200°C, bevorzugt +20°C bis +150°C.

Erfolgt die Polymerisation ionisch, so kann es sich um kationische, bevorzugt jedoch um anionische Polymerisation handeln.

Als Initiatoren kommen metallorganische Verbindungen, wie Diäthylzink oder Diisobutylzink, Naphthalinnatrium, n-Amylnatrium, Cyclopentadienylnatrium, n-Butyllithium oder Triäthylaluminium in Frage. Als Initiatoren wirken ferner Basen, wie Alkalimetallhydroxide, -alkoholate und -amide. Anstelle der bei der radikalischen Polymerisation angewandten Reglern, werden bei der ionischen Polymerisation Stoffe eingesetzt, die mit dem wachsenden Kettenende reagieren; hierzu zählen z.B. Wasser, Alkohole, Säuren und Amine. Die Temperatur bei dieser Reaktionsvariante beträgt von —100°C bis +200°C, bevorzugt —20°C bis +150°C und ist dem Fachmann für den jeweils gewünschten Polymertyp bekannt.

Es ist ferner ebenfalls möglich, als Initiatoren für die Polymerisation Wärme, Licht oder andere energiereiche Strahlung zu verwenden. Die Reaktion läuft dabei nach dem radikalischen Mechanismus ab. Bei der photoinitiierten Polymerisation sind als Katalysatoren z.B. Benzoinäther, Benzilketale, $\omega$-Dialkoxy-acetophenon-Derivate oder aromatische Keton-Amin-Kombinationen geeignet.

Die beschriebenen Methoden eignen sich grundsätzlich sowohol für Homo- wie auch für Copolymeristionsreaktionen. Je nach Wahl der Copolymerisationsparameter erhält man auf diese Weise statistische Copolymere oder auch Blockcopolymere. Für die vorliegende Erfindung ist wesentlich, dass durch den Zusatz von Co-Monomer-Komponenten nur die physikalischen Eigenschaften wie Erweichungspunkt. Löslichkeit u.ä. beeinflusst werden, nicht aber deren Nützlichkeit als Lichtschutzmittel für organisches Material. Währenddem die Copolymerisation zweier Monomerer und die damit verbundenen Effekte besonders gut untersucht worden und dem Fachmann bekannt sind, ist es in gewissen Fällen auch von Nutzen, Polymere aus drei oder mehr polymerisierbaren Verbindungen einzusetzen. Solche weniger bekannte Polymere sind beispielsweise in G. E. Ham, Copolymerization, High Polymers *18* (1964) beschrieben.

Die Verbindungen der Formel I können gemäss der vorliegenden Erfindung als Stabilisatoren für Kunststoffe gegen deren Schädigung durch Einwirkung von Sauerstoff, Wärme und Licht verwendet werden. Beispiele für solche Kunststoffe sind die in der DT—OS 2 456 864 auf den Seiten 12—14 aufgeführten Polymeren.

Von besonderer Bedeutung ist die Stabilisierung von Polyolefinen, Styrolpolymerisaten, Polyamiden und von Polyurethan, für die sich die Verbindungen der Formel I hervorragend eignen, Beispiele hierfür sind Polyäthylen hoher und niedriger Dichte, Polypropylen, Aethylen-Propylen-Copolymerisate, Polystyrol, Styrol-Butadien-Acrylnitril-Tercopolymerisate, Mischungen von Polyolefinen oder von Styrolpolymerisaten, Polyurethane auf Polyäther- oder Polyesterbasis in Form von Lacken, Fäden, Folien, Platten, Filmen, Elastomeren oder Schaumstoffen.

Die homopolymeren oder copolymeren Stabilisatoren werden den Kunststoffen in einer Konzentration von 0,05 bis 5 Gew.-%, berechnet auf das zu stabilisierende Material, zugesetzt. Vorzugsweise werden 0,1 bis 2,5 Gew.-% der Verbindungen, berechnet auf das zu stabilisierende Material, in dieses eingearbeitet.

Die Einarbeitung kann nach der Polymerisation erfolgen, beispielsweise durch Einmischen der Verbindungen und gegebenenfalls weiterer Additive in die Schmelze nach den in der Technik üblichen Methoden, vor oder während der Formgebung, oder auch durch Aufbringen der gelösten oder dispergierten Verbindungen auf das Polymere, gegebenenfalls unter nachträglichem Verdunsten des Lösungsmittels.

Die neuen Verbindungen können auch in Form eines Masterbatches, der diese Verbindungen beispielsweise in einer Konzentration von 2,5 bis 25 Gew.-% enthält, den zu stabilisierenden Kunststoffen zugesetzt werden.

Die Erfindung betrifft daher auch die durch Zusatz von 0,01 bis 5 Gew.-% einer Verbindung der Formel I stabilisierten Kunststoffe, die gegebenenfalls noch andere bekannte und übliche Zusätze enthalten können. Die so stabilisierten Kunststoffe können in verschiedenster Form angewendet werden, z.B. als Folien, Fasern, Bändchen. Profile oder als Bindemittel für Lacke, Klebemittel oder Kitte.

Als weitere Additive, mit denen zusammen die erfindungsgemäss verwendbaren Stabilisatoren eingesetzt werden können, sind beispielsweise zu nennen:

Antioxydantien, wie 2,6-Dialkylphenole, Derivate von alkylierten Hydrochinonen, hydroxylierte Thiodiphenyläther, Alkyliden-bisphenole, O—, N— und S—Benzylverbindungen, hydroxybenzylierte Malonester, Hydroxybenzyl- Aromaten, 2-Triazinverbindungen, Amide der $\beta$ - (3,5 - Di - tert. - butyl - 4 - hydroxyphenyl) - propionsäure, Ester der $\beta$ - (3,5 - Di - tert. - butyl - 4 - hydroxyphenyl) - propionsäure, Ester der $\beta$ - (5 - tert. - Butyl - 4 - hydroxy - 3 - methylphenyl) - propionsäure, Ester der 3,5 - Di - tert. - butyl - 4 - hydroxyphenylessigsäure, Acylaminophenole, Benzylphosphonate, Aminoarylderivate, UV-Absorber und Lichtschutzmittel, wie 2-(2'-Hydroxyphenyl)-benztriazole, 2,4-Bis-(2'-hydroxyphenyl)-6-alkyl-s-triazine, 2-Hydroxybenzophenone, 1,3-Bis-(2'-hydroxybenzoyl)-benzole, Ester von gegebenenfalls substituierten Benzoesäuren, Acrylate, des weiteren Nickelverbindungen, sterisch gehinderte Amine, Oxalsäurediamide, Metalldesaktivatoren, Phosphite, peroxidzerstörende Verbindungen, Polyamidstabilisatoren, basische Co-Stabilisatoren, PVC-Stabilisatoren, Nukleierungsmittel oder sonstige Zusätze, wie z.B. Weichmacher, Gleitmittel, Emulgatoren, Füllstoffe, Russ, Asbest, Kaolin, Talk, Glasfasern, Pigmente, optische Aufheller, Flammschutzmittel, Antistatica.

Beispiele für weitere Additive, mit denen zusammen die erfindungsgemäss verwendbaren Stabilisatoren eingesetzt werden können, finden sich in der DT—OS 2 427 853 auf Seiten 18—24.

Beispiel 1

In einem mit Rückflusskühler, Tropftrichter, Thermometer, $N_2$-Einleitungsrohr und Rührer versehenen Reaktionsgefäss wurden 25 ml reinstes, wasserfreies Toluol vorgelegt und auf 110°C erhitzt. Zu dem vorgelegten Toluol wurde in einer reinen Stickstoffatmosphäre, unter Rühren innerhalb von $2\frac{1}{2}$ Stunden regelmässige die Lösung von 47,8 g 1,2,2,6,6 - Pentamethyl - 4 - methacryloyloxy - piperidin (hergestellt aus 1,2,2,6,6 - Pentamethyl-4-hydroxy - piperidin und Methacrylsäurechlorid in Chloroform in Gegenwart von Triäthylamin, K.p.: 74—75°C/6,67 Pa) und 380 mg $\alpha,\alpha'$-Azoisobutyronitril in 60 ml reinem Toluol getropft. Nach der Zugabe dieser Lösung wurde der Kolbeninhalt weitere 10 Minuten bei 110—113°C gerührt.

Hierauf wurde mit 70 mg 1-Dodecanthiol in 2 ml reinstem Toluol als Polymerisationsregler versetzt und das Polymerisationsgemisch weiter $1\frac{1}{2}$ Stunden bei 110—112°C gerührt.

Die auf 80°C gekühlte Polymerisationslösung wurde hierauf unter kräftigem Turbinieren (Homorex) innerhalb von ca. 5 Minuten in 1,3 Liter Methanol von —20°C eingegossen und die Mischung innerhalb von ca. 30 Minuten langsam auf Raumtemperatur erwärmt. Das harzartig abgeschiedene Polymerisat wurde nach dem Abdekantieren der Methanollösung in wenig Dichlormethan gelöst und nochmals — wie beschrieben — durch Eingiessen in Methanol umgefällt. Das so gereinigte Poly-1,2,2,6,6-pentamethyl-4-methacryloxypiperidin trocknete man bei 80°C im Vakuumschrank. Das erhaltene farblose, leicht pulverisierbare Polymere hat einen Erweichungspunkt von ~ 190°C Mol Gew. ($\bar{M}n$) ≈ 12'000.

Beispiel 2

In einem mit Rückflusskühler, Tropftrichter, Thermometer, $N_2$-Einleitungsrohr und Rührer versehenen Reaktionsgefäss wurden 10 ml reinstes, wasserfreies Toluol vorgelegt und auf 110°C erwärmt. In einer reinen $N_2$-Atmosphäre und unter Rühren wurde innerhalb von 110 Minuten die Lösung von 30 g 1 - Benzyl - 2,2,6,6 - tetramethyl - 4 - acryloyloxypiperidin, 10,4 g frisch destilliertem Styrol und 0,20 g $\alpha,\alpha'$-Azoisobutyronitril in 25 ml reinstem Toluol ins Reaktionsgefäss getropft. Nach der Zugabe dieser Lösung wurde das Polymerisationsgemisch weitere 70 Minuten unter Rühren bei 110—112°C gehalten. Die nach dieser Zeit entstandene, ziemlich viskose, wasserklare Lösung wurde mit 15 ml Toluol verdünnt und für weitere 60 Minuten bei 110—112°C weiter gerührt.

Die auf ca. 70°C gekühlte Polymerisationslösung wuerde hierauf unter kräftigem Turbinieren (Homorex) in 800 ml auf 0°C vorgekühlten Methanols gegossen, wodurch das Polymerisat als feines weisses Pulver ausfiel. Nach weiteren 30 Minuten Turbinieren bei Raumtemperatur wurde das Copolymerisat abfiltriert, mit Methanol gut ausgewaschen und im Vakuumschrank bei 70°C getrocknet. Das so erhaltene farblose Copolymerisat weist einen Erweichungspunkt von 110°C auf.

Das als monomere Komponente eingesetzte 1 - Benzyl - 2,2,6,6 - tetramethyl - 4 - acryloyloxy - piperidin wurde durch Umsetzung von 1 - Benzyl - 2,2,6,6 - tetramethyl - 4 - hydroxypiperidin (Smp.: 156—157°C) mit frisch destilliertem Acrylsäurechlorid in reinem Chloroform bei 0° bis 10°C in Gegenwart von 1,1 Aequivalent wasserfreiem Triäthylamin erhalten. Umkristallisation aus Acetonitril ergab das analysenreine Monomere vom Smp.: 66—68°C.

Beispiel 3

A) Analog dem Beispiel 2 kann aus 1,2,2,6,6-Pentamethyl-4-hydroxypiperidin und Acrylsäurechlorid das 1,2,2,6,6-Pentamethyl-4-acryloyloxypiperidin gewonnen werden. Kp: 52—54°C/2,67 Pa.

B) Das in Verfahren 3A beschriebene Produkt kann auch durch Umesterung eines Acrylsäureesters (z.B. Methyl- oder Aethylester) mit 1,2,2,6,6-Pentamethyl-4-hydroxypiperidin in Gegenwart eines basischen Katalysators (es eignen sich z.B. Tetrabutyl- oder Tetraisopropyl-titanat, Alkaliamid, Aluminium-isopropoxid oder ein Alkalialkoxid) erhalten werden, wobei das sich bildende Alkanol fortlaufend abdestilliert wird. Nach Destillation und Rectifikation besitzt das Produkt die unter A) angegebenen physikalischen Daten.

Beispiele 4 bis 23

Analog der in Beispiel 3A oder 3B beschriebenen Methode wurden folgende polymerisierbare Monomere hergestellt:

| Beispiel | Monomer | physikalische Eigenschaften | Methode gem. Beispiel |
|---|---|---|---|
| 4 | 1-Benzyl-2,2,6,6-tetramethyl-methacryloyloxy-piperidin | Kp: 175°C /4Pa | 1 |
| 5 | 1,2,2,6,6-Pentamethyl-4-acrylamido-piperidin | F: 133–135°C | 3A |
| 6 | 1-n-Butyl-2,2,6,6-tetramethyl-4-acryloyloxy-piperidin | Kp: 96°C /13Pa | 3A, 3B |
| 7 | 2,2,6,6-Tetramethyl-4-(N-n-butyl)-acrylamido-piperidin | Kp: 175–180°C / 6,67Pa | 3A |
| 8 | 1,2,2,6,6-Pentamethyl-4-meth-acrylamido-piperidin | F: 127°C | 3A |
| 9 | 1-Methacryloyloxyäthyl-2,2,6,6-tetramethyl-piperidin | Kp: 130°C / 5,3Pa | 3A, 3B |
| 10 | 1-Acetyl-2,2,6,6-tetramethyl-4-acryloyloxy-piperidin | F: 40–41°C | 3A,B + $Ac_2O$ |
| 11 | 2,2,6,6-tetramethyl-4-acryloyl-oxy-piperidin | F: 55–56°C | 3A, 3B |
| 12 | 1-Allyl-2,2,6,6-tetramethyl-4-acryloyloxy-piperidin | Kp: 77–78°C / 5,3Pa | 3B |
| 13 | 2,5,6-Trimethyl-2,6-diäthyl-4-acryloyloxy-piperidin | Kp: 80°C /1,3Pa | 3B |
| 14 | 1,2,5,6-Tetramethyl-2,6-diäthyl-4-methacryloyloxy-piperidin | Kp: 110°C / 1,3Pa | 3B |
| 15 | 1-Acryloyloxyäthyl-2,2,6,6-tetramethyl-piperidin | Kp: 100°C / 1,3Pa | 3A, 3B |
| 16 | 1-[2'-acryloyloxybutyl]-2,2,6,6-tetramethyl-piperidin | Kp: 145–150°C / 5,3Pa | 3B |
| 17 | 1,3,8-Triaza-2,4-dioxo-3-acryloyloxyäthyl-7,7,8,9,9-pentamethyl-spiro-[4,5]-decan | F: 163–165°C | 3A, 3B |
| 18 | 1,3,8-Triaza-2,4-dioxo-3-n-butyl-7,7,9,9-tetramethyl-8-acryloyloxyäthyl-spirol [4,5]-decan | F: 153–155°C | 3A, 3B |

| Beispiel | Monomer | physikalische Eigenschaften | Methode gem. Beispiel |
|---|---|---|---|
| 19 | 1,3,8-Triaza-2,4-dioxo-3-n-octyl-7,7,9,9-tetramethyl-8-acryloyloxyäthyl-spirol [4,5]-decan | F: 97–98 °C | 3A, 3B |
| 20 | 1,2,2,6,6-Pentamethyl-4-(N-n-propyl)-acrylamido-piperidin | F: 43–44 °C | 3A |
| 21 | 1-Benzyl-2,2,6,6-tetramethyl-4-(N-n-butyl)-acrylamido-piperidin | F: 108–110 °C | 3A |
| 22 | 2,5,6-Trimethyl-2,6-diäthyl-4-(N-methyl)-acrylamido-piperidin | KP: 110 °C / 0,13Pa | 3A |
| 23 | 1,2,2,6,6-Pentamethyl-4-malein-imido-piperidin<br>(hergestellt aus 1,1,2,2,6,6-Pentamethyl-4-amino-piperidin und Maleinimid) | F: 116–117 °C | |

Beispiel 24

In einem mit Rückflusskühler, Tropftrichter, Thermometer, Gaseinleitungsrohr und Ankerrührer versehenen Reaktionsgefäss wurden (nachdem das Reaktionsgefäss mit Argon vollständig gespült worden war) 445 g destilliertes 4-Acryloyloxy-1,2,2,6,6-pentamethyl-piperidin und 100 mg Dodecylmercaptan in 1500 ml reinem Benzol vorgelegt und die Lösung rasch auf 76°C erwärmt. Bei dieser Temperatur tropfte man unter Rühren innerhalb von 45 Min. sehr regelmässig die Lösung von 1,5 g $\alpha,\alpha'$-Azoisobutyronitril (gelöst in 50 ml reinem Benzol) zu, wobei die Innentemperatur auf max. 80° anstieg und die Polymerisationswärme durch den Rückflusskühler abgeführt wurde. Anschliessend wurde die Polymerisationslösung noch für weitere 22 Std. bei 75—76°C weiter gerührt.

Aus der nunmehr etwas viskosen, immer noch vollständig farblosen Reaktionslösung wurden hierauf am Rotationsverdampfer im Wasserstrahlvakuum ca. 1000 ml Benzol abdestilliert. Die konzentrierte, viskose Polymerlösung goss man nun unter kräftigem Turbinieren langsam in 3,6 Liter Methanol von 0°C, wobei bei dieser Temperatur vorerst nur eine geringe Polymerfällung entsteht. Durch langsames Erwärmen der Methanol-Lösung auf 25—28°C wurde die Fällung vervollständigt. Nach ca. 30 Min. Turbinieren wurde das überstehende Methanol dekantiert und die Polymerfällung wiederholt mit wenig Methanol nachgewaschen, wieder dekantiert und der Bodenkörpoer schliesslich in Dichlormethan gelöst und im Wasserstrahlvakuum am Rotationsverdampfer vom Lösungsmittel möglichst vollständig befreit. Das pulvrigspröde Polymere wurde im Vakuumtrockenschrank bei 80° noch vollständig getrocknet. Das so erhaltene, leicht pulverisierbare, farblose polymere 4-Acryloyloxy-1,2,2,6,6-pentamethyl-piperidin weist einen Erweichungspunkt von ~115°C und ein mittleres Molekulargewicht ($\overline{M}_n$) von 29'000 auf.

Beispiele 25 bis 82

Analog den Beispielen 1,2 oder 24 wurden Polymere bzw. Copolymere aus den folgenden Monomeren hergestellt.

| Beispiel | Monomer (Beispiel Nr.) | Polymer /Copolymer<br>Ep = Erweichungspunkt<br>$\overline{Mn}$ = Molgewicht<br>R = Regler,<br>I = Initiator |
|---|---|---|
| 25 | 3 | Ep: ~115°C<br>$\overline{Mn}$: ~13:000<br>analog Bsp 1 |
| 26 | 3 | Ep: ~112°C<br>$\overline{Mn}$: ~ 8'500<br>analog Bsp 1 |
| 27 | 3 | Ep: ~115°C<br>$\overline{Mn}$: ~ 90:000<br>analog Bsp 24 in Benzol<br>R: – |
| 28 | 3 | Ep: ~115°C<br>$\overline{Mn}$: ~ 33'600<br>analog Bsp 24 |
| 29 | 3 | Ep: ~ 110°C<br>$\overline{Mn}$: ~ 6'400<br>analog Bsp 24 in Tetrahydrofuran |
| 30 | 3 | Ep: ~112–115°C<br>$\overline{Mn}$: ~22'200<br>analog Bsp 24 in Toluol-<br>R: –<br>I: Dibenzoylperoxid |
| 31 | 3 | Ep: ~ 115°C<br>$\overline{Mn}$: ~ 38'600<br>analog Bsp 24<br>R: 30 mg /Mol Monomer |
| 32 | 3 | Ep: ~115°C<br>$\overline{Mn}$: ~3'660<br>analog Bsp 24 |

| Beispiel | Monomer (Beispiel Nr.) | Polymer / Copolymer |
|---|---|---|
| 33 | 3 | Ep: ~ 115°C<br>$\overline{Mn}$: ~ 22'200<br>analog Bsp 24<br>R: 90 mg/Mol Monomer |
| 34 | 2 | Ep: 135–140°C<br>analog Bsp 1 |
| 35 | 4 | Ep; 210°C<br>$\overline{Mn}$: ~ 3'000<br>analog Bsp 1 |
| 36 | 6 | Ep: 120°C<br>$\overline{Mn}$: ~ 7'000<br>analog Bsp 1 |
| 37 | 10 | Ep: 125°C<br>$\overline{Mn}$: ~ 5'900<br>analog Bsp 24<br>R: – |
| 38 | 11 | Ep: 90–95°C<br>$\overline{Mn}$: 13'200<br>analog Bsp 24 |
| 39 | 12 | Ep: ~ 200°C<br>analog Bsp 24 |
| 40 | 13 | Ep: ~ 90°C<br>$\overline{Mn}$: ~ 10'000<br>analog Bsp 24<br>R: – |
| 41 | 14 | Ep: ~ 200°C<br>$\overline{Mn}$: ~ 7'600<br>analog Bsp 24<br>R: – |
| 42 | 9 | Ep: ~ 145°C<br>$\overline{Mn}$: ~ 7'000<br>analog Bsp 1 |
| 43 | 15 | Ep: ~ 70°C<br>$\overline{Mn}$: ~ 4'000<br>analog Bsp 1<br>R: – |

| Beispiel | Monomer (Beispiel Nr.) | Polymer / Copolymer |
|---|---|---|
| 44 | 16 | Ep: ∼ 85°C<br>$\overline{Mn}$: 3'000<br>analog Bsp 24 |
| 45 | 17 | Ep: ∼165°C<br>$\overline{Mn}$: ∼14'000<br>analog Bsp 24<br>R: – |
| 46 | 18 | Ep: ∼160°C<br>$\overline{Mn}$: ∼7'000<br>analog Bsp 24 |
| 47 | 19 | Ep: ∼125°C<br>$\overline{Mn}$: ∼8'200<br>analog Bsp 24<br>R: – |
| 48 | 5 | Ep: 195–200°C<br>$\overline{Mn}$: ∼6'000<br>analog Bsp 24 in Tetrahydrofuran<br>R: – |
| 49 | 8 | Ep: ∼220°C<br>$\overline{Mn}$: ∼15'000<br>analog Bsp 24<br>R: – |
| 50 | 7 | Ep: ∼250°C<br>analog Bsp 24 |
| 51 | 20 | Ep: ⩾250°C<br>$\overline{Mn}$: ∼20'000<br>analog Bsp 24 |
| 52 | 22 | Ep: ∼220°C<br>$\overline{Mn}$: ∼6'000<br>analog Bsp 24<br>R: – |
| 53 | 21 | Ep: ∼220°C<br>$\overline{Mn}$: ∼12'600<br>analog Bsp 24<br>R: – |

| Beispiel | Monomer (Beispiel Nr.) | Polymer /Copolymer |
|---|---|---|
| 54 | 1 Teil 3 +<br>1 Teil Styrol | Ep: ∼ 110°C<br>$\overline{Mn}$: ∼ 6'700 |
| 55 | 2 Teile 3 +<br>1 Teil Styrol | Ep: 115–120°C<br>$\overline{Mn}$: ∼ 14'400 |
| 56 | 1 Teil 1 +<br>1 Teil Styrol | Ep: 155–160°C<br>$\overline{Mn}$: ∼ 9'700 |
| 57 | 0.9 Teile 2 +<br>1 Teil Styrol | Ep: 110–115°C<br>$\overline{Mn}$: ∼ 2'200 |
| 58 | 1 Teil 2 +<br>1 Teil Styrol | Ep: 110–115°C<br>$\overline{Mn}$: ∼ 3'000 |
| 59 | 3 Teile 3 +<br>2 Teile Acrylsäure-äthylester | Ep: ∼ 80°C<br>$\overline{Mn}$: ∼ 30'000 |
| 60 | 2 Teile 3 +<br>1 Teil Acrylsäure-t.butylester | Ep: ∼ 105°C<br>$\overline{Mn}$: ∼ 150'000 |
| 61 | 2 Teile 3 +<br>1 Teil Acrylsäure-äthylester | Ep: ∼ 96°C<br>$\overline{Mn}$: ∼ 33'000 |
| 62 | 5 Teile 1 +<br>1 Teile Acrylsäure-äthylester | Ep: ∼ 165°C<br>$\overline{Mn}$: ∼ 19'600 |
| 63 | 1 Teil 1 +<br>1 Teil Methacryl-säure-äthylester | Ep: ∼ 145°C<br>$\overline{Mn}$: ∼ 6'500 |
| 64 | 6 Teile 3 +<br>5 Teile Acrylonitril | Ep: ∼ 120°C<br>$\overline{Mn}$: ∼ 29'800 |
| 65 | 1 Teil 3 +<br>1 Teil N-Vinyl-pyrrolidon-2 | Ep: 145–150°C<br>$\overline{Mn}$: ∼ 8'200 |
| 66 | 2 Teile 3 +<br>1 Teile N-Vinyl-pyrrolidon-2 | Ep: 120–125°C<br>$\overline{Mn}$: 15'300 |

| Beispiel | Monomer (Beispiel Nr.) | Polymer /Copolymer |
|---|---|---|
| 67 | 2 Teile 2 + 1 Teil N-Vinyl-pyrrolidon-2 | Ep: 155–160°C |
| 68 | 1 Teil 1 + 1 Teil Vinylacetat | Ep: ~165°C<br>$\overline{Mn}$: ~88'000 |
| 69 | 2 Teile 3 + 1 Teil Acrylsäure-t.butylester + 1 Teil N-Vinyl-pyrrolidon-2 | |
| 70 | 2 Teile 3 + 1 Teil N-Vinyl-pyrrolidon-2 | |
| 71 | 2 Teile 3 + 1 Teil Acrylsäure-2,3-epoxypropylester | Ep: ~80°C |
| 72 | 4 Teile 3 + 1 Teil Acrylsäure-2-hydroxyäthylester | |
| 73 | 3 Teile 3 + 1 Teil Acrylsäure | |
| 74 | 2 Teile 3 + 1 Teil Acrylsäure-t.butylamid | Epi: ~115°C<br>$\overline{Mn}$: ~ 9'400 |
| 75 | 3 Teile 3 + 2 Teile Maleinimid | Ep: ~ 210°C |
| 76 | 1 Teil 3 + 1 Teil N-Aethyl-maleinimid | Ep: ~ 145°C<br>$\overline{Mn}$: ~ 15'700 |
| 77 | 1 Teil 3 + 0,85 Teile N-Benzyl-maleinimid | Ep: 135–138°C<br>$\overline{Mn}$: ~ 8'400 |
| 78 | 1 Teil 3 + 1 Teil N-Phenyl-maleinimid | Ep: ~ 205°C<br>$\overline{Mn}$: ~ 14'500 |
| 79 | 1 Teil 3 + 1 Teil 23 | Ep: ~ 200–205°C<br>$\overline{Mn}$: ~ 20'000 |

| Beispiel | Monomer (Beispiel Nr.) | Polymer /Copolymer |
| --- | --- | --- |
| 80 | 1 Teil 23 + 1 Teil Acrylsäure-aethylester | Ep: ~155°C $\overline{Mn}$: ~10'200 |
| 81 | 6 Teile 3 + 5 Teile 23 | Ep: ~205°C $\overline{Mn}$: 14'600 |
| 82 | 6 Teile 3 + 5 Teile N-Benzyl-maleinimid | Ep: ~165°C $\overline{Mn}$: ~15'500 |
| 83 | 1 Teil 3 + 1 Teil 1 | Ep: ~165°C $\overline{Mn}$: ~28'900 |
| 84 | 1 Teil 3 + 1 Teil 20 | Ep: ~150°C $\overline{Mn}$: ~11'500 |

Beispiel 85

Lichtschutzwirkung in PP-Fasern

1000 Teile unstabilisiertes Polypropylenpulver (Schmelzindex ~ 18) werden in einem Trommelmischer mit 1 Teil Pentaerithrytyl-tetrakis [3 - (3,5 - di - tert - butyl - 4 - hydroxyphenyl) - propionat] und mit 3 Teilen der in der Tabelle angeführten Lichtschutzmittel gemischt und anschliessend in einem Extruder bei 220°C extrudiert und granuliert. Das erhaltene Granulat wird in einer Labor-Schmelzspinnanlage bei einer maximalen Temperatur von 270°C und einer Geschwindigkeit von 300 m/Minute zu einem 570/12 denier Multifilament versponnen. Dieses wird verstreckt und gezwirnt mittels einer Streckzwirnmaschine. Das Streckverhältnis ist 1:5.6 und die Zwirnzahl 15/Meter, so dass schliesslich Multifilamente 120/12 denier erhalten werden. Diese Multifilamente werden auf weissen Karton montiert und im Xenotest 1200 belichtet.

Als Mass der Schutzwirkung gilt die Belichtungszeit bis 50% Reissfestigkeitsverlust.

Die Ergebnisse sind in der Tabelle zusammengefasst.

| Additiv. Nr. | Stunden bis 50% Restreissfestigkeit |
| --- | --- |
| keines | 215 |
| 32 | 2850 |
| 58 | 500 |

**Patentansprüche**

1. Organische Polymere enthaltend als Lichtschutzmittel seitenständige N-heterocyclische Ringe tragende homopolymere Verbindungen mit der wiederkehrenden Struktureinheit der Formel I

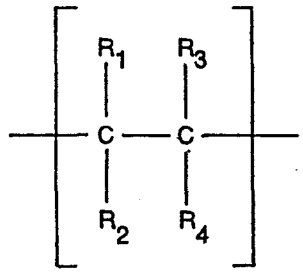

(I),

worin

$R_1$ eine einen N-heterocyclischen Ring enthaltende Gruppe der Formeln II, III, IV und V

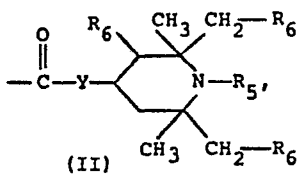

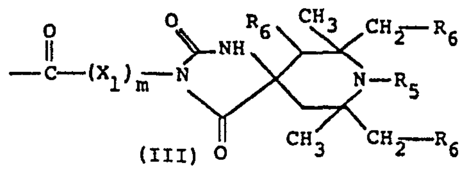

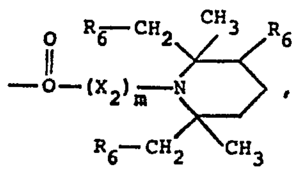

(IV)

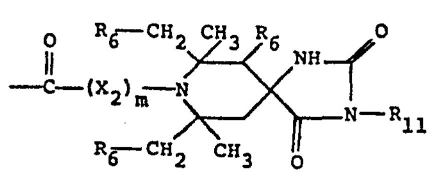

(V)

bedeuten, worin

$R_5$ Wasserstoff, Oxyl-Sauerstoff, $C_1$—$C_{18}$-Alkyl, $C_3$—$C_8$-Alkenyl, $C_3$—$C_6$-Alkinyl, $C_7$—$C_{12}$-Aralkyl, $C_2$—$C_{21}$-Alkoxyalkyl, eine aliphatische Acylgruppe mit 1—4 C-Atomen oder eine der Gruppen $—CH_2COOR_7$ oder $—COOR_8$ ist, wobei

$R_7$ $C_1$—$C_{12}$-Alkyl, $C_3$—$C_8$-Alkenyl, Phenyl, $C_7$—$C_8$-Aralkyl oder Cyclohexyl bedeutet, und

$R_8$ $C_1$—$C_{12}$-Alkyl, Phenyl, Benzyl oder Cyclohexyl ist, und

$R_6$ Wasserstoff oder $C_1$—$C_4$-Alkyl bedeutet, und

Y —O— oder $—N—R_9$ ist, wobei

$R_9$ Wasserstoff, $C_1$—$C_{12}$-Alkyl, $C_7$—$C_{12}$-Aralkyl oder Cyclohexyl ist, und

$X_1$ eine Gruppe der Formel $—O—CH(R_{10})—CH_2—$ (VI) ist, worin

$R_{10}$ Wasserstoff, Methyl, Aethyl, Phenoxymethyl oder Phenyl bedeutet, und

n 0 oder 1 ist, und

$X_2$ eine Gruppe der Formel VI, worin $R_{10}$ die oben angegebene Bedeutung hat, oder eine Gruppe der Formel $—O—CH_2—CH(OH)—CH_2—$ (VII) ist, und

m 0 oder 1 ist, und

$R_{11}$ $C_1$—$C_{18}$-Alkyl, gegebenenfalls durch $C_1$—$C_4$-Alkyl oder $C_1$—$C_4$-Alkoxy substituiertes Cyclohexyl, Phenyl, oder Benzyl bedeutet, und

$R_2$ Wasserstoff oder $C_1$—$C_4$-Alkyl ist, und

$R_1$ und

$R_3$ falls $R_2$ und $R_4$ Wasserstoff sind, zusammen eine Gruppe der Formel VIII

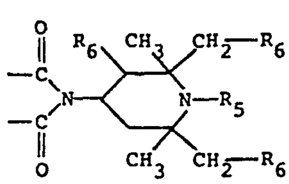

(VIII) bilden, worin

$R_5$ und

$R_6$ die oben angegebene Bedeutung hat, und

$R_3$ Wasserstoff oder $C_1$—$C_2$-Alkyl ist, und

$R_4$ Wasserstoff oder Methyl bedeutet,

oder deren Copolymere mit mindestens eine polymerisierbare Doppelbindung enthaltenden Verbindung, wobei das Molverhältnis der Komponente der Formel I zu der Co-Komponente bis zu 1:10 beträgt und wobei das Molekulargewichts-Zahlenmittel der homo- oder copolymeren Lichtschutzmittel 500 bis 150'000, vorzugsweise 500 bis 50'000 beträgt.

2. Organische Polymere enthaltend homopolymere Verbindungen gemäss Anspruch 1, der Formel I, worin

$R_1$ eine Gruppe der Formeln II, III, IV oder V bedeutet, worin
$R_5$ Wasserstoff, $C_1$—$C_{12}$-Alkyl, $C_3$—$C_5$-Alkenyl, Propargyl, $C_7$—$C_8$-Aralkyl, Acetyl oder $C_2$—$C_{10}$-Alkoxyalkyl bedeutet, und
$R_6$ Wasserstoff oder Methyl bedeutet, und
Y —O— oder

$$\begin{array}{c} | \\ —N—R_9 \end{array}$$

ist, worin
$R_9$ Wasserstoff, $C_1$—$C_{12}$-Alkyl oder Benzyl ist, und
$X_1$ eine Gruppe der Formel VI, worin $R_{10}$ Wasserstoff oder Methyl ist, bedeutet, und
$R_{11}$ $C_1$—$C_{12}$-Alkyl oder Benzyl ist, und
$R_2$ Wasserstoff oder $C_1$—$C_2$-Alkyl bedeutet, oder
$R_1$ und
$R_3$ falls $R_2$ und $R_4$ Wasserstoff sind, zusammen eine Gruppe der Formel VIII bilden,
oder deren Copolymere mit mindestens eine polymerisierbare Doppelbindung enthaltenden Verbindungen.

3. Organische Polymere enthaltend homopolymere Verbindungen gemäss Anspruch 2, der Formel I, worin

$R_1$ eine Gruppe der Formeln II oder IV bedeutet, worin
$R_5$ Wasserstoff, $C_1$—$C_8$-Alkyl, Benzyl oder Acetyl ist, und
Y —O— oder

$$\begin{array}{c} | \\ —N—R_9 \end{array}$$

bedeutet, worin
$R_9$ Wasserstoff oder $C_1$—$C_8$-Alkyl ist, und
$X_2$ eine Gruppe der Formel VI oder VII ist, worin
$R_{10}$ Wasserstoff, Methyl oder Aethyl ist, und
$R_2$ Wasserstoff oder Methyl bedeutet, oder
$R_1$ und $R_3$ falls $R_2$ und $R_4$ Wasserstoff sind, zusammen eine Gruppe der Formel VIII sind, und
$R_3$ Wasserstoff oder Methyl ist, und
$R_4$ Wasserstoff bedeutet,
oder deren Copolymere mit mindestens eine polymerisierbare Doppelbindung enthaltenden Verbindungen.

4. Organische Polymere, enthaltend homopolymere Verbindungen gemäss Anspruch 3, der Formel I, wobei

$R_5$ Wasserstoff, $C_1$—$C_4$-Alkyl, Benzyl oder Acetyl bedeutet, und
$R_6$ Wasserstoff ist, und
Y —O— oder

$$\begin{array}{c} | \\ —N—R_9 \end{array}$$

bedeutet, worin
$R_9$ Wasserstoff oder $C_1$—$C_4$-Alkyl ist, und
$X_2$ eine Gruppe der Formeln VI oder VII ist, worin
$R_{10}$ Wasserstoff oder Methyl ist, und
oder deren Copolymere mit Styrol, Acrylnitril, einem Acryl- oder einem Methacrylsäureester, einem Vinylester, einem Vinyläther, oder einem Acryl- oder Methacrylamid.

5. Organische Polymere enthaltend homo- oder copolymere Verbindungen gemäss Anspruch 4, der Formel I, wobei

$R_5$ Wasserstoff, Methyl oder Acetyl ist, und
$X_2$ eine Gruppe der Formeln VI oder VII ist, worin
$R_{10}$ Wasserstoff ist, und
$R_3$ und
$R_4$ Wasserstoff bedeutet, und
$R_2$ Wasserstoff oder Methyl ist,

6. Organische Polymere, enthaltend homo- oder copolymere Verbindungen gemäss Anspruch 1 der Formel I, worin

$R_1$ eine Gruppe der Formel II ist und
Y —O— ist.

7. Organische Polymere gemäss Anspruch 1, dadurch gekennzeichnet, dass das Molekulargewichts-Zahlenmittel der homo- oder copolymeren Lichtschutzmittel 1000 bis 20.000 beträgt.

8. Organische Polymere gemäss Anspruch 1, enthaltend Copolymere aus einer Verbindung der Formel I und einem Acrylsäure- oder Methacrylsäure-methyl, -äthyl oder -butylester, einem Maleinimid, N-Vinylpyrrolidon oder einem Acrylsäureglycidylester, wobei das Molverhältnis der Komponente der Formel I zur Co-Komponente bis zu 1:10 beträgt.

9. Organische Polymere, enthaltend homo- oder copolymere Verbindungen gemäss Anspruch 1 der Formel I, worin

$R_{10}$ Wasserstoff oder Methyl ist,

10. Homo- oder copolymere Verbindungen, gemäss Anspruch 1 der Formel I, worin

$R_1$ eine Gruppe der Formel III, IV oder V ist, und die übrigen Symbole die in Anspruch 1 angegebene Bedeutung haben, mit einem Molekulargewichts-Zahlenmittel von 500 bis 150'000, vorzugsweise 500 bis 50'000.

11. Copolymere aus einer Verbindung der Formel I, gemäss Anspruch 1 und einer mindestens eine polymerisierbare Doppelbindung enthaltende Co-Komponente, wobei das Verhältnis der Verbindung der Formel I zur Co-Komponente bis zu 1:10 beträgt und deren Molekulargewichts-Zahlenmittel 500 bis 150'000, vorzugsweise 500 bis 50'000 beträgt.

12. Copolymere gemäss Anspruch 11, dadurch gekennzeichnet, dass die Co-Komponente ein Acrylsäure- oder Methacrylsäure-methyl, -äthyl, oder -butylester, ein Maleinimid, N-Vinylpyrrolidon, ein Acryl- oder Methacrylsäureamid oder Acrylsäureglycidylester ist.

13. Homo- oder copolymere Verbindungen gemäss Anspruch 1, der Formel I, worin $R_1$ und $R_3$ zusammen eine Gruppe der Formel VIII bedeuten und deren Molekulargewichts-Zahlenmittel 500 bis 150'000, vorzugsweise 500 bis 50'000 beträgt.

**Claims**

1. An organic polymer containing as light stabiliser a homopolymeric compound which carries N-heterocyclic rings in a side position and has the recurring structural unit of the formula I

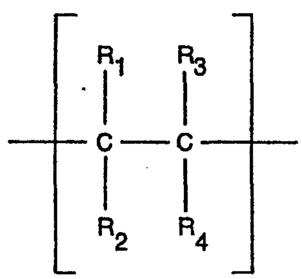

(I)

in which

$R_1$ is a group, containing an N-heterocyclic ring, of the formulae II, III IV and V

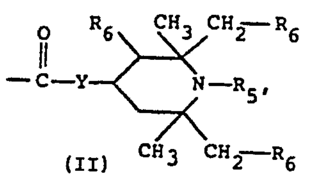

(II)

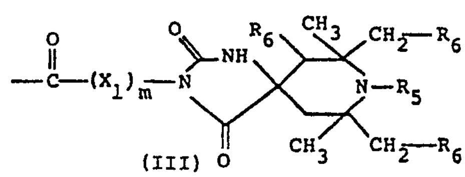

(III)

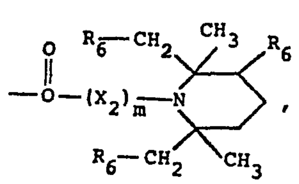

(IV)

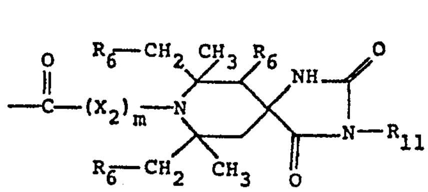

(V)

in which

$R_5$ is hydrogen, oxyl, $C_1$—$C_{18}$ alkyl, $C_3$—$C_8$ alkenyl, $C_3$—$C_6$ alkynyl, $C_7$—$C_{12}$ aralkyl, $C_2$—$C_{21}$ alkoxyalkyl, an aliphatic acyl group having 1—4 C atoms or one of the groups —$CH_2COOR_7$ or —$COOR_8$, in which

$R_7$ is $C_1$—$C_{12}$ alkyl, $C_3$—$C_8$ alkenyl, phenyl, $C_7$—$C_8$ aralkyl or cyclohexyl and
$R_8$ is $C_1$—$C_{12}$ alkyl, phenyl, benzyl or cyclohexyl, and
$R_6$ is hydrogen or $C_1$—$C_4$ alkyl and
Y is —O— or

$$-\overset{|}{N}-R_9,$$

in which
$R_9$ is hydrogen, $C_1$—$C_{12}$ alkyl, $C_7$—$C_{12}$ aralkyl or cyclohexyl, and
$X_1$ is a group of the formula —O—CH($R_{10}$)—$CH_2$— (VI), in which $R_{10}$ is hydrogen, methyl, ethyl, phenoxymethyl or phenyl, and n is 0 or 1, and
$X_2$ is a group of the formula VI, in which
$R_{10}$ is as defined above, or a group of the formula —O—$CH_2$—CH(OH)—$CH_2$— (VII), and
m is 0 or 1, and
$R_{11}$ is $C_1$—$C_{18}$ alkyl or is cyclohexyl, phenyl or benzyl which are unsubstituted or substituted by $C_1$—$C_4$ alkyl or $C_1$—$C_4$ alkoxy, and
$R_2$ is hydrogen or $C_1$—$C_4$ alkyl, and, if $R_2$ and $R_4$ are hydrogen,
$R_1$ and
$R_3$ together form a group of the formula VIII

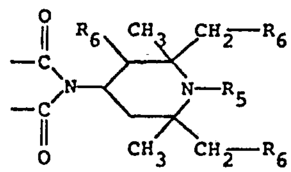

(VIII)

in which
$R_5$ and
$R_6$ are as defined above and
$R_3$ is hydrogen or $C_1$—$C_2$ alkyl and $R_4$ is hydrogen or methyl, or a copolymer thereof with a compound containing at least one polymerisable double bond, the molar ratio of the component of the formula I to the co-component being up to 1:10 and the average molecular weight of the homopolymeric or copolymeric light stabiliser being 500 to 150,000, preferably 500 to 50,000.

2. An organic polymer containing a homopolymeric compound according to claim 1 of the formula I, in which
$R_1$ is a group of the formulae II, III, IV or V, in which
$R_5$ is hydrogen, $C_1$—$C_{12}$ alkyl, $C_3$—$C_5$ alkenyl, propargyl, $C_7$—$C_8$ aralkyl, acetyl or $C_2$—$C_{10}$ alkoxyalkyl and
$R_6$ is hydrogen or $C_1$—$C_8$ alkyl, and
Y is —O— or

$$-\overset{|}{N}-R_9,$$

in which
$R_9$ is hydrogen, $C_1$—$C_{12}$ alkyl or benzyl, and
$X_1$ is a group of the formula VI, in which
$R_{10}$ is hydrogen or methyl, and
$R_{11}$ is $C_1$—$C_{12}$ alkyl or benzyl, and
$R_2$ is hydrogen or $C_1$—$C_2$ alkyl, or, if $R_2$ and $R_4$ are hydrogen,
$R_1$ and
$R_3$ together form a group of the formula VIII, or a copolymer thereof with a compound containing at least one polymerisable double bond.

3. An organic polymer containing a homopolymeric compound according to claim 2, of the formula I, in which
$R_1$ is a group of the formulae II or IV, in which
$R_5$ is hydrogen, $C_1$—$C_8$ alkyl, benzyl or acetyl, and
Y is —O— or

$$-\overset{|}{N}-R_9,$$

in which

$R_9$ is hydrogen of $C_1$—$C_8$ alkyl, and

$X_2$ is a group of the formula VI or VII, in which

$R_{10}$ is hydrogen, methyl or ethyl, and $R_2$ is hydrogen or methyl or, if $R_2$ and $R_4$ are hydrogen, $R_1$ and

$R_3$ together are a group of the formula VIII, and $R_3$ is hydrogen or methyl and $R_4$ is hydrogen, or a copolymer thereof with a compound containing at least one polymerisable double bond.

4. An organic polymer containing a homopolymeric compound according to claim 3 of the formula I, in which

$R_5$ is hydrogen, $C_1$—$C_4$ alkyl, benzyl or acetyl and

$R_6$ is hydrogen, and

Y is —O— or

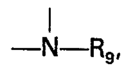

in which

$R_9$ is hydrogen or $C_1$—$C_4$ alkyl, and $X_2$ is a group of the formulae VI or VII, in which

$R_{10}$ is hydrogen or methyl, or a copolymer thereof with styrene, acrylonitrile, an acrylate or methacrylate, a vinyl ester, a vinyl ether or an acrylamide or methacrylamide.

5. An organic polymer containing a homopolymeric or copolymeric compound according to claim 4 of the formula I, wherein $R_5$ is hydrogen, methyl or acetyl, and $X_2$ is a group of the formula VI or VII, in which $R_{10}$ is hydrogen, and $R_3$ and $R_4$ are hydrogen and $R_2$ is hydrogen or methyl.

6. An organic polymer containing a homopolymeric or copolymeric compound according to claim 1 of the formula I, wherein $R_1$ is a group of the formula II and Y is —O—.

7. An organic polymer according to claim 1, wherein the average molecular weight of the homopolymeric or copolymeric light stabiliser is 1000 to 20,000.

8. An organic polymer according to claim 1, containing a copolymer of a compound of the formula I and a methyl, ethyl or butyl acrylate or methacrylate, a maleimide, N-vinylpyrrolidone or a glycidyl acrylate, the molar ratio of the component of the formula I to the co-component being up to 1:10.

9. An organic polymer containing a homopolymeric or copolymeric compound according to claim 1, of the formula I, in which $R_{10}$ is hydrogen or methyl.

10. A homopolymeric or copolymeric compound according to claim 1 of the formula I, in which $R_1$ is a group of the formula III, IV or V and the other symbols are as defined in claim 1, said compound having an average molecular weight of 500 to 150,000 preferably from 500 to 50,000.

11. A copolymer of a compound of the formula I according to claim 1, and a co-component which contains at least one polymerisable double bond, the ratio of the compound of the formula I to the co-component being up to 1:10, said copolymer having an average molecular weight of 500 to 150,000, preferably from 500 to 50,000.

12. A copolymer according to claim 11, wherein the co-component is a methyl, ethyl or butyl acrylate or methacrylate, a maleimide, N-vinylpyrrolidone or an acrylamide or methacrylamide or glycidyl acrylate.

13. A homopolymeric or copolymeric compound according to claim 1, of the formula I, in which $R_1$ and $R_3$ together are a group of the formula VIII, said compound having an average molecular weight of 500 to 150,000, preferably from 500 to 50,000.

**Revendications**

1. Polymères organiques contenant, comme stabilisants à la lumière, des composés homopolymères porteurs de noyaux hétérocycliques azotés latéraux et comportant des motifs répétés répondant à la formule I

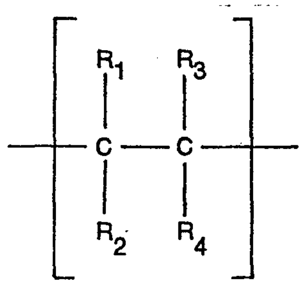

(I)

dans laquelle

$R_1$ représente un radical contenant un noyau hétérocyclique azoté et répondant à l'une des formules II, III, IV et V

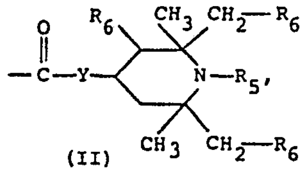

(II)

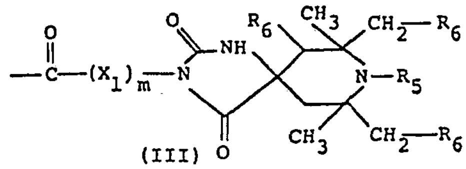

(III)

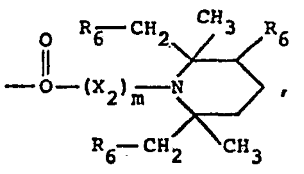

(IV)

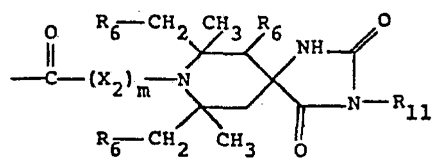

(V)

dans lesquelles

$R_5$ représente l'hydrogène, un oxygène oxyle, un alkyle en $C_1$—$C_{18}$, un alcényle en $C_3$—$C_8$, un alcynyle en $C_3$—$C_6$, un aralkyle en $C_7$—$C_{12}$, un alcoxy-alkyle en $C_2$—$C_{21}$, un acyle aliphatique contenant de 1 à 4 atomes de carbone ou un radical répondant à l'une des formules —$CH_2COOR_7$ et —$COOR_8$ dans lesquelles

$R_7$ représente un alkyle en $C_1$—$C_{12}$, un alcényle en $C_3$—$C_8$, un phényle, un aralkyle en $C_7$ ou $C_8$ ou un cyclohexyle et

$R_8$ représente un alkyle en $C_1$—$C_{12}$, un phényle, un benzyle ou un cyclohexyle,

$R_6$ représente l'hydrogène ou un alkyle en $C_1$—$C_4$,

Y représente —O— ou un groupe

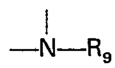

dans lequel

$R_9$ représente l'hydrogène, un alkyle en $C_1$—$C_{12}$, un aralkyle en $C_7$—$C_{12}$ ou un cyclohexyle,

$X_1$ représente un radical —O—CH($R_{10}$)—$CH_2$— (VI) dans lequel

$R_{10}$ représente l'hydrogène, un méthyle, un éthyle, un phénoxyméthyle ou un phényle,

n est égal à 0 ou à 1,

$X_2$ représente un radical (VI) dans lequel $R_{10}$ a la signification précédemment donnée, ou un radical —O—$CH_2$—CH(OH)—$CH_2$— (VII),

m est égal à 0 ou à 1 et

$R_{11}$ représente un alkyle en $C_1$—$C_{18}$, ou représente un radical cyclohexyle, phényle ou benzyle éventuellement porteur d'un alkyle en $C_1$—$C_4$ ou d'un alcoxy en $C_1$—$C_4$,

$R_2$ représente l'hydrogène ou un alkyle en $C_1$—$C_4$,

$R_1$ et

$R_3$, dans le cas où $R_2$ et $R_4$ désignent chacun l'hydrogène, forment ensemble un radical de formule VIII

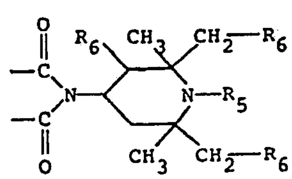

(VIII)

dans lequel $R_5$ et $R_6$ ont les significations précédemment données,

$R_3$ représente l'hydrogène ou un alkyle en $C_1$ ou $C_2$ et

$R_4$ représente l'hydrogène ou un méthyle,

ou leurs copolymères avec au moins un composé contenant une double liaison polymérisable, le rapport molaire de la composante de formule I à la co-composante pouvant aller jusqu'à 1:10 et le poids moléculaire moyen en nombre des stabilisants à la lumière homopolymères ou copolymères étant compris entre 500 et 150.000, de préférence entre 500 et 50.000.

2. Polymères organiques contenant des composés homopolymères de formule I selon la revendication 1, dans lesquels:

$R_1$ représente un radical de formule II, III, IV ou V dans lequel

$R_5$ représente l'hydrogène, un alkyle en $C_1$—$C_{12}$, un alcényle en $C_3$—$C_5$, un propargyle, un aralkyle en $C_7$ ou $C_8$, un acétyle ou un alcoxyalkyle en $C_2$—$C_{10}$,
$R_6$ représente l'hydrogène ou un méthyle,
Y représente —O— ou un radical

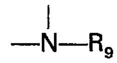

dans lequel
$R_9$ représente l'hydrogène, un alkyle en $C_1$—$C_{12}$ ou un benzyle,
$X_1$ représente un radical de formule VI dans lequel $R_{10}$ désigne l'hydrogène ou un méthyle, et
$R_{11}$ représente un alkyle en $C_1$—$C_{12}$ ou un benzyle,
$R_2$ représente l'hydrogène ou un alkyle en $C_1$ ou $C_2$, ou
$R_1$ et
$R_3$, lorsque $R_2$ et $R_4$ sont chacun l'hydrogène, forment ensemble un radical de formule VIII,
ou leurs copolymères avec des composés contenant au moins une double liaison polymérisable.

3. Polymères organiques contenant des composés homopolymères de formule I selon la revendication 2, dans lesquels:
$R_1$ représente un radical de formule II ou IV dans lequel
$R_5$ représente l'hydrogène, un alkyle en $C_1$—$C_8$, un benzyle ou un acétyle,
Y représente —O— ou un radical

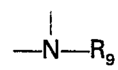

dans lequel
$R_9$ représente l'hydrogène ou un alkyle en $C_1$—$C_8$,
$X_2$ représente un radical de formule VI ou VII dans lequel
$R_{10}$ représente l'hydrogène ou un radical méthyle ou éthyle,
$R_2$ représente l'hydrogène ou un méthyle, ou
$R_1$ et
$R_3$, dans le cas où $R_2$ et $R_4$ représentent chacun l'hydrogène, forment ensemble un radical de formule VIII,
$R_3$ représente l'hydrogène ou un méthyle, et
$R_4$ représente l'hydrogène,
ou leurs copolymères avec des composés contenant au moins une double liaison polymérisable.

4. Polymères organiques contenant des composés homopolymères de formule I selon la revendication 3, dans lesquels:
$R_5$ représente l'hydrogène, un alkyle en $C_1$—$C_4$, un benzyle ou un acétyle,
$R_6$ représente l'hydrogène,
Y représente —O— ou un radical

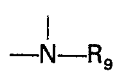

dans lequel
$R_9$ représente l'hydrogène ou un alkyle en $C_1$—$C_4$,
$X_2$ représente un radical de formule VI ou VII dans lequel
$R_{10}$ représente l'hydrogène ou un méthyle,
ou leurs copolymères avec le styrène, l'acrylonitrile, un ester de l'acide acrylique, un ester de l'acide méthacrylique, un ester vinylique, un éther vinylique, un acrylamide ou un méthacrylamide.

5. Polymères organiques contenant des composés homopolymères ou copolymères de formule I selon la revendication 4, dans lesquels:
$R_5$ représente l'hydrogène, un méthyle ou un acétyle,
$X_2$ représente un radical de formule VI ou VII dans lequel
$R_{10}$ représente l'hydrogène,
$R_3$ et
$R_4$ représentent chacun l'hydrogène et
$R_2$ représente l'hydrogène ou un méthyle.

6. Polymères organiques contenant des composés homopolymères ou copolymères de formule I selon la revendication 1, dans lesquels:
$R_1$ représente un radical de formule II et
Y représente —O—.

7. Polymères organiques selon la revendication 1, caractérisés en ce que le poids moléculaire moyen en nombre des stabilisants à la lumière homopolymères ou copolymères est compris entre 1.000 et 20.000.

8. Polymères organiques selon la revendication 1, qui contiennent des copolymères provenant d'un composé de formule I et d'un ester méthylique, éthylique ou butylique de l'acide acrylique ou de l'acide méthacrylique, d'un maléimide, de la N-vinyl-pyrrolidone ou d'un acrylate de glycidyle, le rapport molaire de la composante de formule I à la co-composante pouvant aller jusqu'à 1:10.

9. Polymères organiques contenant des composés homopolymères ou copolymères de formule I selon la revendication 1 dans lesquels $R_{10}$ représente l'hydrogène ou un méthyle.

10. Composés homopolymères de formule I selon la revendication 1, dans lesquels $R_1$ représente un radical de formule III, IV ou V et les autres symboles ont les significations données à la revendication 1, avec un poids moléculaire moyen en nombre de 500 à 150.000, de préférence de 500 à 50.000.

11. Copolymères constitués d'un composé de formule I selon la revendication 1 et d'une co-composante contenant au moins une double liaison polymérisable, le rapport du composé de formule I à la co-composante pouvant aller jusqu'à 1:10 et le poids moléculaire moyen en nombre desdits copolymères étant compris entre 500 et 150.000, de préférence entre 500 et 50.000.

12. Copolymères selon la revendication 11, caractérisés en ce que la co-composante est un ester méthylique, éthylique ou butylique de l'acide acrylique ou de l'acide méthacrylique, un maléimide, la N-vinyl-pyrrolidone, un acrylamide, un méthacrylamide ou l'acrylate de glycidyle.

13. Composés homopolymères ou copolymères de formule I selon la revendication 1 dans lesquels $R_1$ et $R_3$ forment ensemble un radical de formule VIII et dont le poids moléculaire moyen en nombre est compris entre 500 et 150.000 de préférence entre 500 et 50.000.